# EUROPÄISCHE PATENTSCHRIFT

(11) **EP 3 328 479 B1**
(45) Veröffentlichungstag und Bekanntmachung des Hinweises auf die Patenterteilung: **27.05.2026**
(21) Anmeldenummer: 16756598.5
(22) Anmeldetag: 29.07.2016
(51) Int. Cl.: A61M 25/10, A61F 2/04, A61F 2/856, A61F 2/82, A61F 2/958

(54) **BALLONKATHETER**
BALLOON CATHETER
CATHÉTER À BALLONNET

(30) Priorität: 29.07.2015 DE 102015112390
(43) Veröffentlichungstag der Anmeldung: 06.06.2018
(73) Patentinhaber: Bentley InnoMed GmbH, 72379 Hechingen (DE)
(72) Erfinder: OBRADOVIC, Milisav, 79539 Lörrach (DE); BREGULLA, Rainer, 72336 Balingen (DE)
(74) Vertreter: Hoefer & Partner Patentanwälte mbB
(86) Internationale Anmeldenummer: PCT/EP2016/068184
(87) Internationale Veröffentlichungsnummer: WO 2017/017259

(56) Entgegenhaltungen:
- WO-A1-97/17101
- DE-T2- 3 779 539
- US-A1- 2002 120 320
- US-A1- 2006 265 041
- US-A1- 2014 276 585
- US-A1- 2014 277 062

## Beschreibung

Die Erfindung betrifft einen Ballonkatheter für TIPS-Prozeduren, mit einem gestuften Ballon, einer Zuleitung im Katheter zum Ballon, die es erlaubt, den Ballon mit Druck zu beaufschlagen, und einem zentralen Lumen für einen Führungsdraht.

Ballonkatheter werden seit vielen Jahren zum Aufweiten von Stents in Gefäßen eingesetzt. Zur Aufweitung von Stents wird ein Stent auf den Ballonkatheter aufgekrimpt und mit Hilfe des Ballonkatheters am gewünschten Implantationsort dilatiert und platziert. Anschließend wird der Ballonkatheter ohne den Stent aus dem Gefäß entfernt.

Stents werden mit Hilfe von Ballonkathetern auch in Verbindungen zwischen zwei Blutgefäßen (sog. Shunts) eingesetzt, etwa in der Leber bei Anlage eines TIPS (Transjugulärer Intrahepatischer Portosystemischer Shunt) bei Verengungen der Pfortader oder Bluthochdruck in der Leber.

Bei der Angioplastie werden Ballonkatheter eingesetzt, um ein verengtes Gefäß mechanisch aufzuweiten und dort gebildete Plaques an die Gefäßwand zu drücken.

Ein besonderes Problem tritt auf, wenn im Bereich von Gefäßabzweigungen neben dem Hauptast auch die Abzweigung mit einem Stent versehen werden muss. In diesem Fall wird zunächst ein mit einer Fenestrierung versehener Stent in den Hauptast verbracht und dort so implantiert, dass das Fenster im Bereich
der Abzweigung angeordnet ist. Sodann wird ein weiterer Stent in die Abzweigung eingebracht, dort dilatiert und durch Aufweiten an den Stent im Hauptast angepasst. Dies fordert in der Regel mehrere separate Schritte, insbesondere dann, wenn sich das abzweigende Gefäß in seinem Verlauf verengt und damit eine abgestufte Aufweitung vorgenommen werden muss. Hinzu kommt die Anpassung des Stents im Seitenast an das Fenster und den Verlauf des Stents im Hauptast.

Für diese Anpassung ist es möglich, abgestuft mit mehreren Ballonen unterschiedlichen Durchmessers vorzugehen, die nacheinander eingeführt werden. Eingesetzt wird aber auch ein sogenannter "Ballon im Ballon", bei dem zwei Ballone so aneinander gekoppelt sind, dass sie separat mit Druck beaufschlagt und für die unterschiedliche Aufweitung eingesetzt werden können. Nachteilig ist der Aufwand beim Einsatz mehrerer separater Ballone.

Aus den Druckschriften US2014/277062A1, US2006/265041A1, DE3779539T2, WO97/17101A1, US2014/276585A1 und US2002/120320A1 sind verschiedene Katheter mit gestuften Ballons bekannt

Aufgabe der Erfindung ist die Bereitstellung eines Ballonkatheters, mit dem Stents in abzweigenden Gefäßen oder Shunts präzise platziert werden können und die Bereitstellung eines Ballonkatheters für TIPS-Prozeduren.

Diese Aufgabe wird mit einem Ballonkatheter gemäß Anspruch 1 gelöst, bei dem die Segmente jeweils Einzelballone sind, die unmittelbar an benachbarte Einzelballone angrenzen und die an ihren Stirnseiten punktuell über Schweißpunkte miteinander verbunden sind, und dass der proximale Ballon kugelförmig ausgebildet ist, wobei der distale Ballon einen kleineren Durchmesser aufweist als der proximale Ballon.

Der erfindungsgemäße Ballonkatheter weist einen Katheterschaft, einen gestuften Ballon, ein Lumen, um den Ballon mit Druck zu beaufschlagen sowie ein Lumen für einen Führungsdraht auf. Der Ballon selbst ist gestuft, dergestalt, dass wenigstens ein Bereich einen gegenüber einem anderen Bereich
erweiterten Durchmesser hat. Stufung bedeutet in diesem Zusammenhang, dass die einzelnen Segmente, etwa der proximale und der distale Bereich, mit einer Stufe ineinander übergehen, wobei die Abstufung in einem relativ engen Bereich erfolgt.

Die Ballone der erfindungsgemäßen Ballonkatheter haben zwei oder mehr Bereiche mit unterschiedlichem Durchmesser. So kann beispielsweise der proximale Bereich einen größeren Durchmesser aufweisen, als der distale Bereich, was für die Platzierung für Stents in Seitenästen eines Hauptgefäßes von Vorteil ist.

Darüber hinaus können erfindungsgemäße Ballonkatheter auch mehrere Abstufungen über die gesamte Länge aufweisen, was beispielsweise bei sich verengenden Gefäßen von Vorteil ist, oder aber einen zentralen Bereich mit einem gegenüber dem proximalen und distalen Bereich reduzierten Durchmesser. Die letztgenannte Variante ist beispielsweise für die Platzierung von Stents in Shunts geeignet, etwas bei der TIPS-Anlage, wo es darauf ankommt, den Stent präzise in den Shunt einzupassen und gleichzeitig nicht zu weit zu expandieren, um so eine Durchflussregulierung zu erreichen.

Der erfindungsgemäße Ballonkatheter kann zum einen zur Erweiterung von Gefäßen verwandt werden, ohne dass dabei Stents platziert werden, zum anderen aber auch zum Platzieren von Stents. Dazu werden die Stents auf den Ballon aufgekrimpt, wobei die Stents sowohl über die gesamte Ballonlänge reichen können, oder auch nur über einen Teilbereich. So wird beispielsweise bei Platzierung eines Stents in einer Gefäßabzweigung der Stent auf dem Ballon so aufgekrempt, dass er in den Bereich der Abstufung hineinragt und bei der Expansion des Ballon an diesem Ende trompetenförmig erweitert wird und somit an die Wandung des Hauptgefäßes oder eines in einem Hauptgefäß platzierten Stents anpassen kann.

Die Bereiche des Ballons des erfindungsgemäßen Ballonkatheters stellen einzelne Segmente dar, die den Stufen entsprechen. Diese Segmente können miteinander in Verbindung stehen oder voneinander durch Wandungen getrennt sein.

Der Ballon hat beispielsweise einen erweiterten proximalen und einen reduzierten distalen Bereich. Der distale Bereich ist eher schlank gehalten. Er kann einen gleichmäßigen Durchmesser über seine Länge aufweisen, sich aber auch zum distalen Ende des Katheters hin weiter verschlanken, um eine Anpassung an sich verengende Seitenäste zu ermöglichen.

Der proximale Bereich des Ballons im erfindungsgemäßen Ballonkatheter weist einen gegenüber dem proximalen Bereich deutlich erweiterten Durchmesser auf. Insbesondere ist der Durchmesser um etwa 50 bis 100 % erweitert.

Zwischen dem proximalen und dem distalen Bereich des Ballons kann sich ein mittlerer Bereich befinden, der einen Durchmesser aufweist, der zwischen dem des proximalen und des distalen Bereichs liegt. In diesem Fall hat der Ballon eine dreifache Stufung. Ebenso ist eine vierfache Stufung möglich, wobei die Durchmesser der einzelnen Segmente von proximal nach distal abnehmen.

In der Regel hat der Ballon in dieser Variante im proximalen Bereich einen Durchmesser (in expandiertem Zustand) von 5 bis 14 mm, im distalen Bereich von 2 bis 6 mm.

Hat der Ballon einen proximal erweiterten Bereich, weist er an seinen Flanken einen relativ steilen Anstieg auf, der vorzugsweise auf beiden Seiten, d. h. der Anstieg vom Katheterschaft einerseits und der Anstieg vom proximal gelegenen Teil des Ballons andererseits, gleichmäßig ausgebildet ist. Zweckmäßigerweise beträgt der Anstieg 45 bis 75° (nicht beansprucht), bezogen auf die Katheterachse. Ein steiler Anstieg der erweiterten Zone ist positiv für die trompetenförmige Aufweitung des Stents im Eingangsbereich der Abzweigung und für die Anpassung an den im Hauptast verlegten Stent bzw. an das Hauptgefäß, von dem die Abzweigung ausgeht.

Der Ballon des erfindungsgemäßen Ballonkatheters kann somit in mehrere Segmente unterteilt sein, wobei jedes Segment eine separate Zuleitung zur Druckbeaufschlagung aufweist. Dabei sind die einzelnen Segmente jeweils Einzelballone, die unmittelbar an benachbarte Einzelballone angrenzen. In diesem Fall sind die Einzelballone zweckmäßigerweise miteinander verbunden.

Die einzelnen Segmente entsprechen zweckmäßigerweise den vorstehend genannten proximalen, distalen und ggf. mittleren Bereichen, also den einzelnen Stufen. Werden Einzelballone verwandt, sind diese an den Berührungspunkten, d. h. dort, wo sich die Abstufungen befinden, miteinander verklebt oder erfindungsgemäß punktuell miteinander verschweißt. Die Verbindung der einzelnen Einzelballone ist wichtig für eine gleichmäßige Expansion.

Bei der Anwendung des in mehrere Segmente oder Einzelballone unterteilten erfindungsgemäßen Ballonkatheters mit erweitertem proximalen Bereich wird zur Platzierung des Stents insbesondere zunächst der distale Bereich dilatiert, danach der mittlere Bereich, sofern vorhanden, und zuletzt der proximale Bereich, der die größte Aufweitung erfordert und bei dem der Stent an die Fenestrierung des Stents im Hauptast oder an die Form der Gabelung im Bereich der Abzweigung angepasst werden muss. Handelt es sich um einen einfachen Ballon ohne Unterteilung in einzeln dilatierbare Segmente, erfolgt die Dilatation gleichmäßig über die gesamte Länge.

Grundsätzlich ist es möglich, den Ballonkatheter mit einem Außenballon zu umgeben, der eng an den darunter liegenden inneren Ballon oder Einzelballonen anliegt. Der Außenballon hat in diesem Fall gemäß einer Variante eine Sicherheitsfunktion, d. h. er wird nicht separat dilatiert, sondern erweitert sich mit der Expansion des darunter liegenden Ballons oder von dessen Segmenten oder Ballonen. Alternativ ist es möglich, auch den Außenballon zu dilatieren, um eine Vorexpansion zu bewirken und dann die Endexpansion über den innenliegenden Ballon zu bewirken. In jedem Fall sind die Konturen des außenliegenden Ballons und des innenliegenden Ballon oder der innenliegenden Ballonsegmente oder Ballone aufeinander abgestimmt.

Der erfindungsgemäße Ballonkatheter wird auf übliche Art und Weise hergestellt, Auch die Materialien sind für diesen Bereich übliche Materialien. Der Unterschied zum Stand der Technik liegt allein im Design der Ballone.

Für die Ballone können übliche Materialien verwandt werden. Vorzugsweise wird für den inneren Ballon ein begrenzt aufdehnbares Material verwandt (non-compliant), etwa Polyamid 12, PET, Nylon, und für den äußeren Ballon ein gut aufdehnbares Material (compliant oder semi-compliant), etwa Silikongummi, Pebax, PA 11 oder eine Mischung aus Pebax und PA 11.

Die Erfindung wird durch die beiliegenden Zeichnungen näher erläutert. Es zeigen:
- Fig. 1:: Eine Gesamtansicht eines Ballonkatheters;
- Fig. 2:: eine Schnittzeichnung durch den Ballonkatheter von Figur 1;
- Fig. 3:: eine zweite Variante eines Ballonkatheters im Schnitt; und
- Fig. 4:: eine Schnittzeichnung eines Doppelballons für TIPS-Prozeduren.

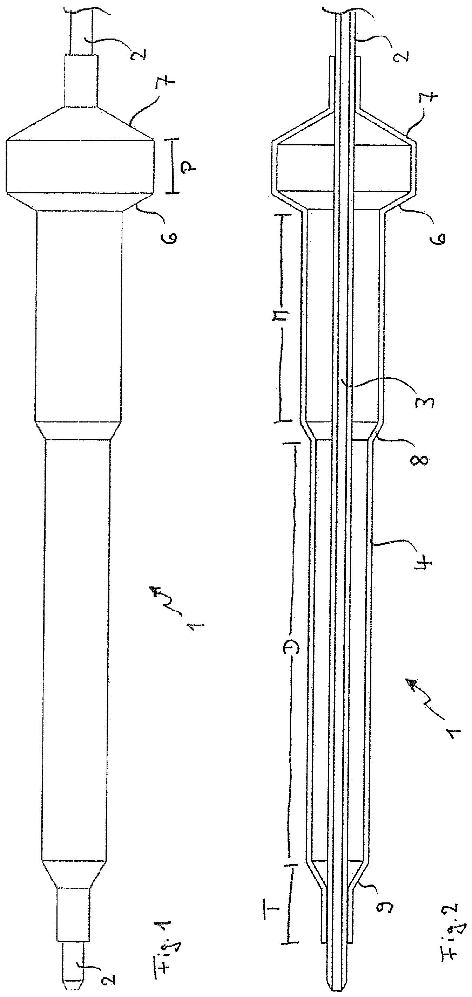

Figur 1 zeigt einen Ballonkatheter 1 mit dem deutlich erweiterten proximalen Bereich P mit steilen Flanken 7 zum Katheter hin und 6 zum distalen Bereich D hin, den relativ schlanken distalen Bereich D, der distal abgestuft auf den Katheterdurchmesser abnimmt. Der Katheter 2 führt durch das Ballonkonstrukt 4 hindurch und endet distal vom Ballonkonstrukt 4.

Für den Gebrauch ist auf den Ballonkatheter ein Stent aufgekrimpt, der durch die Expansion der Ballone aufgeweitet und in einem Blutgefäß platziert wird. Die Darstellung zeigt den Katheter 1 mit expandiertem Ballon.

Figur 2 zeigt eine Schnittzeichnung durch den Ballonkatheter 1 gemäß Figur 1 mit dem Katheter 2, einem freien Lumen 3 für einen Führungsdraht zum Platzieren des Katheters und dem Ballon 4.

Der Ballon 4 ist in den proximalen Bereich P, den distalen Bereich D, den mittleren Bereich M und den terminalen Bereich T differenziert.

Der proximale Bereich P ist gegenüber dem distalen Bereich D deutlich erweitert. Der mittlere Bereich M bleibt in seinen Durchmessern, hinter dem proximalen Bereich P zurück, hat aber einen größeren Durchmesser als der distale Bereich D. Die Übergänge vom proximalen Bereich P zum mittleren Bereich M und vom mittleren Bereich M zum distalen Bereich D wie auch in dem terminalen Bereich T werden durch relativ steile Flanken 6, 8 und 9 gebildet. Die Flanke 6 ist entscheidend für die Anpassung eines Stents an die Fenestrierung eines Stents im Hauptast bzw. für die Anpassung an die Gefäßwandung im Hauptast bei der Platzierung des Stents.

Im terminalen Bereich T verschlankt sich der Ballon und schließt dichtend vor dem Ende des Katheters 2 ab. Die Kanäle, die zum Befüllen der Ballone mit dem Fluid dienen, sind konventionell und in der Zeichnung nicht dargestellt.

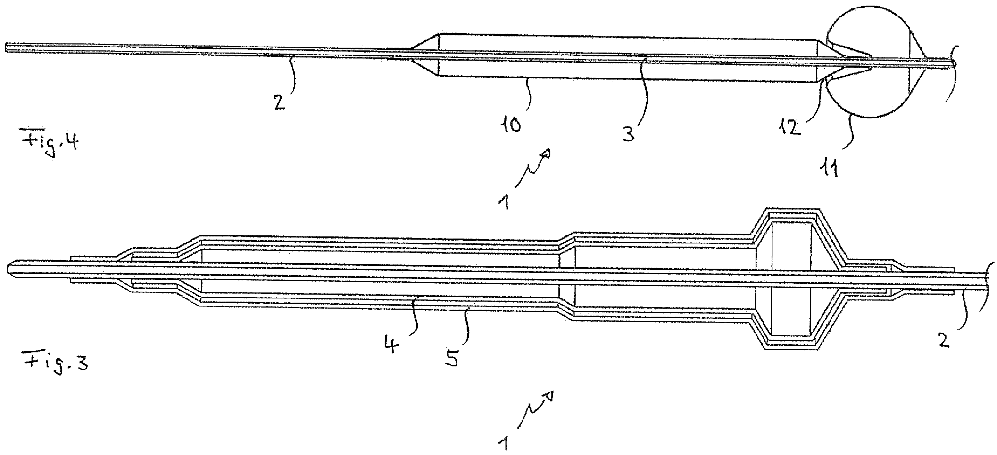

Figur 3 zeigt eine Variante des Doppelballons, bei dem der Ballon 4 durch einen äußeren Ballon 5 umgeben ist. Der äußere Ballon 5 gibt eine größere Sicherheit und erlaubt, sofern eine separate Expansion über ein weiteres Lumen vorhanden ist, eine präzisere und gezieltere Aufweitung und Anpassung eines Stents an den Verlauf des Gefäßes. Im Übrigen entspricht der Ballon 1 gemäß Figur 3 der Darstellung in Figur 2.

Figur 4 zeigt einen erfindungsgemäßen Ballonkatheter 1 für TIPS-Prozeduren, der einen proximalen Ballon 11 mit erweitertem Durchmesser und einen distalen Ballon 10 kleineren Durchmessers aufweist. Beide Ballone sind separat expandierbar, wie dies für TIPS-Prozeduren in der Leber erforderlich ist. Die beiden Ballone sind über Klebe-oder Schweißpunkte 12 miteinander verbunden, so dass sie in expandiertem Zustand eine Einheit bilden. Nicht dargestellt sind die Lumina, die der separaten Befüllung der Ballone dienen. Dargestellt ist das Lumen 3 für einen Führungsdraht zur Platzierung der Vorrichtung 1.

Es versteht sich, dass es zahlreiche Abwandlungen im Bereich der Gestaltung des proximalen und distalen Bereichs gibt. Gemäß der Erfindung ist der proximale Bereich kugelförmig ausgebildet. Der distale Bereich ist mit gleichem Durchmesser dargestellt (nicht beansprucht), jedoch ist es ohne Weiteres möglich, hier eine weitere Abstufung oder Verschlankung zum terminalen Ende des Katheters hin vorzusehen. So kann beispielsweise der distale Bereich über seine Länge zum terminalen Ende hin eine Reduktion des Durchmessers von 40% erfahren, wobei diese Verschlankung kontinuierlich oder stufenförmig erfolgen kann.

## Patentansprüche

1. Gestufter Ballonkatheter für TIPS-Prozeduren mit einem Ballon (4), Zuleitungen im Katheter zum Ballon (4), die es erlauben, den Ballon (4) mit Druck zu beaufschlagen, und einem zentralen Lumen (3) für einen Führungsdraht, wobei der Ballon (4) in mehrere Segmente unterteilt ist und jedes Segment eine separate Zuleitung zur Druckbeaufschlagung aufweist und wobei der Ballon im expandierten Zustand wenigstens zwei Bereiche (P, D) mit unterschiedlichem Durchmesser aufweist und diese Bereiche (P, D) mit einer Stufe ineinander übergehen, **dadurch gekennzeichnet, dass** die Segmente jeweils Einzelballone sind, die unmittelbar an benachbarte Einzelballone angrenzen und die an ihren Stirnseiten punktuell über Schweißpunkte miteinander verbunden sind, und dass der proximale Ballon kugelförmig ausgebildet ist, wobei der distale Ballon einen kleineren Durchmesser aufweist als der proximale Ballon.

2. Ballonkatheter nach Anspruch 1, **dadurch gekennzeichnet, dass** der proximale Bereich (P) des Ballons (4) gegenüber dem distalen Bereich (D) um 50 % bis 100 % erweitert ist.

3. Ballonkatheter nach einem der Ansprüche 1 oder 2, **dadurch gekennzeichnet, dass** der erweiterte Bereich einen steilen Anstieg der Flanken (6, 7) aufweist.

4. Ballonkatheter nach einem der vorstehenden Ansprüche, **dadurch gekennzeichnet, dass** der Ballon (4) von einem Außenballon (5) umgeben ist.

5. Ballonkatheter nach einem der vorstehenden Ansprüche mit aufgekrimptem Stent.

## Claims

1. Stepped balloon catheter for TIPS procedures, being provided with a balloon (4), supply lines in the catheter leading to the balloon (4), which allow the balloon (4) to be pressurized, and a central lumen (3) for a guidewire, wherein the balloon (4) is subdivided into several segments, and each segment having a separate supply line for pressurization purposes and wherein the balloon in its expanded state comprises at least two areas (P, D) of different diameter, with these areas (P, D) merging into one another by forming a step, **characterized in that** each of the segments are single balloons that are directly adjacent to neighboring single balloons and are connected to each other via weld spots at their end faces, and **in that** the proximal balloon has a spherical-like shape, wherein the distal balloon has a smaller diameter than the proximal balloon.

2. Balloon catheter according to claim 1, **characterized in that** the proximal area (P) of the balloon (4) has an enlarged diameter by 50% to 100% relative to the distal region (D).

3. Balloon catheter according to claim 1 or 2, **characterized in that** the enlarged area has steeply rising flanks (6, 7).

4. Balloon catheter according to any one of the preceding claims, **characterized in that** the balloon (4) is surrounded by an external balloon (5).

5. Balloon catheter according to any one of the preceding claims, **characterized in that** a stent is crimped on the balloon.

## Revendications

1. Cathéter à ballonnet étagé pour des procédures TIPS avec un ballonnet (4), des lignes d'alimentation dans le cathéter vers le ballonnet (4) permettant de soumettre le ballonnet (4) à l'action d'une pression, et une lumière centrale (3) pour un fil-guide, dans lequel le ballonnet (4) est divisé en plusieurs segments et chaque segment présente une conduite d'arrivée séparée pour l'application de pression et dans lequel le ballonnet présente dans l'état expansé au moins deux zones (P, D) de diamètre différent et lesdites zones (P, D) se fondent l'une dans l'autre par un étage, **caractérisé en ce que** les segments sont chacun des ballonnets individuels qui jouxtent directement des ballonnets individuels adjacents et qui sont ponctuellement reliés entre eux sur leurs faces frontales par des points de soudure, et que le ballonnet proximal est de forme sphérique, dans lequel le ballonnet distal présente un diamètre plus petit que le ballonnet proximal.

2. Cathéter à ballonnet selon la revendication 1, **caractérisé en ce que** la région proximale (P) du ballonnet (4) est élargie de 50 % à 100 % par rapport à la région distale (D).

3. Cathéter à ballonnet selon l'une quelconque des revendications 1 ou 2, **caractérisé en ce que** la zone élargie présente une montée abrupte des flancs (6, 7).

4. Cathéter à ballonnet selon l'une quelconque des revendications précédentes, **caractérisé en ce que** le ballonnet (4) est entouré d'un ballonnet extérieur (5).

5. Cathéter à ballonnet selon l'une quelconque des revendications précédentes avec une endoprothèse sertie.
